# EUROPÄISCHE PATENTSCHRIFT

(11) **EP 0 446 479 B1**
(45) Veröffentlichungstag und Bekanntmachung des Hinweises auf die Patenterteilung: **06.04.1994**
(21) Anmeldenummer: 90125326.0
(22) Anmeldetag: 22.12.1990
(51) Int. Cl.: A61M 25/01, A61M 5/50, A61M 5/32, A61B 19/02

(54) **Aufnahmevorrichtung für eine Stahlkanüle**
Retaining device for a steel cannula
Dispositif pour retenir une canule d'acier

(30) Priorität: 16.03.1990 DE 4008392
(43) Veröffentlichungstag der Anmeldung: 18.09.1991
(73) Patentinhaber: VYGON GMBH & CO KG, D-52070 Aachen (DE)
(72) Erfinder: Heiliger, Raymund, Dr., W-5120 Herzogenrath (DE)
(74) Vertreter: Bauer, Hubert, Dipl.-Ing.

(56) Entgegenhaltungen:
- EP-A- 0 322 129
- CH-A- 538 288
- GB-A- 1 267 161

## Beschreibung

Die Erfindung betrifft eine Aufnahmevorrichtung aus einer mit einem Längsschlitz versehenen Schutzhülse zur Unterbringung einer Stahlkanüle. Diese ist an ihrem proximalen Ende mit einem Ansatz versehen, an den ein radial vorstehendes Betätigungselement angeformt ist.

Eine derartige Aufnahmevorrichtung ist aus der GB-A-1 267 161 bekannt.

Mit derartigen Aufnahmevorrichtungen wird insbesondere eine verletzungsfreie Entsorgung von benutzten Stahlkanülen bezweckt, die je nach ihrer durch die Benutzung erfolgten Kontamination eine beachtliche Infektionsquelle darstellen können.

Eine bekannte Aufnahmevorrichtung besteht aus einer im Querschnitt rechteckigen, kastenförmigen Schutzhülse, die mit einem Längsschlitz versehen ist, der bis zu einem Abstand vor dem Eintrittsende der Schutzhülse reicht. Ein gleichfalls kastenförmiger Schlitten umschließt die Schutzhülse und ist durch an die Schutzhülse angeformte Anschläge zwischen zwei Endpositionen verschiebbar. Ein innenseitig am Schlitten angeformter Steg weist durch den Schlitz in die kastenförmige Schutzhülse hinein und bildet mit dem Ansatz der Stahlkanüle ein Stück. So wie der Schlitten in Längsrichtung der Schutzhülse relativ zu dieser verschoben wird, taucht die mit dem Schlitten somit starr verbundene Stahlkanüle zunehmend in die Schutzhülse ein, bis sich in einer arretierbaren Endstellung des Schlittens die Stahlkanüle gänzlich innerhalb der Schutzhülse befindet.

Mit der bekannten Aufnahmevorrichtung läßt sich zwar eine verletzungsfreie Entsorgung einer benutzten Stahlkanüle gewährleisten. Die dazu vorgesehene Schutzhülse und insbesondere der mit dem Ansatz der Stahlkanüle einstückig ausgebildete Schlitten erfordern komplizierte Formen für diese Teile und verteuern die Aufnahmevorrichtung insgesamt erheblich.

Der Erfindung liegt die Aufgabe zugrunde, eine Aufnahmevorrichtung vorzuschlagen, die nicht nur kostengünstig herzustellen, sondern auch für konventionelle Stahlkanülen verwendbar ist, ohne daß deren Ansatz mit besonders ausgebildeten Betätigungsmitteln zur Überführung der Stahlkanüle in die Aufnahmevorrichtung ausgestattet werden muß.

Zur Lösung dieser Aufgabe wird von einer Aufnahmevorrichtung der im Oberbegriff des Anspruchs 1 genannten gattungsgemäßen Art ausgegangen, welche erfindungsgemäß die im kennzeichnenden Teil desselben angegebenen Merkmale aufweist.

Der Innendurchmesser der erfindungsgemäßen Schutzhülse ist so dem Außendurchmesser des zylindrischen Ansatzstücks der auf die Stahlkanüle aufgeschobenen Kunststoffkanüle angepaßt, daß sich unter geringfügiger Aufweitung des Längsschlitzes der Schutzhülse diese auf das Ansatzstück aufklemmen läßt. Der bis zum aufgeklemmten Ende der Schutzhülse reichende Längsschlitz ermöglicht es, daß bereits beim Aufklemmen der Schutzzhülse auf das Ansatzstück der Kunststoffkanüle das Betätigungselement in den aufgeweiteten Anfangsbereich des Schlitzes gelangt und bei weiterhin unbehinderter Erfaßbarkeit den Schlitz entlang verschiebbar ist. Mit zunehmender Verschiebung des Betätigungselements, das vorzugsweise die einfache Form einer mit zwei Fingerkuppen erfaßbaren, radial vom Ansatzstück der Stahlkanüle abstehenden Platte aufweist, wird zwangsläufig auch die Stahlkanüle zunehmend in die Schutzhülse hinein verschoben, bis sich die Stahlkanüle gänzlich innerhalb der Schutzhülse befindet. Sodann kann die Schutzhülse vom Ansatzstück der Kunststoffkanüle abgezogen und mit der eingehüllten Stahlkanüle gefahrlos entsorgt werden.

Um zur Erleichterung der Einführung des Betätigungselements in den Schlitz nicht auf dessen Aufweitung allein durch das Aufklemmen der Schutzhülse auf das Ansatzstück der Kunststoffkanüle angewiesen zu sein, sieht eine Ausgestaltung der Erfindung vor, den Schlitz am auf das Ansatzstück aufsetzbaren Ende der Schutzhülse durch dreiecksförmige Aussparungen im Hülsenmantel zu verbreitern.

Obschon durch die elastische Aufweitbarkeit des Schlitzes der Schutzhülse das Betätigungselement in jeder Position zwischen den Randkanten des Schlitzes zangenartig gegen eine unbeabsichtigte Verlagerung gehalten ist und somit auch eine in die Schutzhülse hineingezogene Stahlkanüle darin verbleibt, läßt sich eine unbeabsichtigte Freigabe der Stahlkanüle nach einer besonders vorteilhaften Ausgestaltung der Erfindung noch sicherer verhindern. Dazu weist der Schlitz in einem Abstand vom auf das Ansatzstück aufgesetzten Ende der Schutzhülse, der mindestens dem Abstand zwischen der Spitze der Stahlkanüle und dem Betätigungselement entspricht, eine radiale Verbreiterung auf, in welche das Betätigungselement einrastbar ist.

Die erfindungsgemäße Aufnahmevorrichtung eignet sich nicht nur zur sicheren Unterbringung einer benutzten Stahlkanüle, sondern ist ebenso hervorrragend geeignet, einem aus einer Stahl- und einer Kunststoffkanüle bestehenden Kanülenset bis zu dessen Einsatz als Schutzvorrichtung zu dienen. Zu diesem Zweck sieht eine weitere Ausgestaltung der Erfindung schließlich noch vor, daß der Innendurchmesser der Schutzhülse an seinem dem auf das Ansatzstück aufgesetzten Ende gegenüberliegenden, gleichfalls offenen Ende dem Außendurchmesser eines zylindrischen Ansatzteils der auf die Stahlkanüle aufgeschobenen Kunststoffkanüle entspricht, welches dem distalen Ende der Kunststoffkanüle zugewandt ist.

In der Zeichnung ist ein Ausführungsbeispiel einer erfindungsgemäßen Aufnahmevorrichtung dargestellt. Es zeigen:
- Fig. 1: eine Stahlkanüle und darauf aufgeschobene Kunststoffkanüle mit einer Aufnahmevorrichtung in einer Seitenansicht teilweise im Schnitt;
- Fig. 2: eine teilweise in die Aufnahmevorrichtung hineingeschobene Stahlkanüle in einer Seitenansicht nach Benutzung;
- Fig. 3: eine teilweise in die in einem Längsschnitt dargestellte Aufnahmevorrichtung hineingeschobene Stahlkanüle nach Benutzung;
- Fig. 4: die Stahlkanüle und die Aufnahmevorrichtung in einem Schnitt nach der Linie IV - IV der Fig. 2.

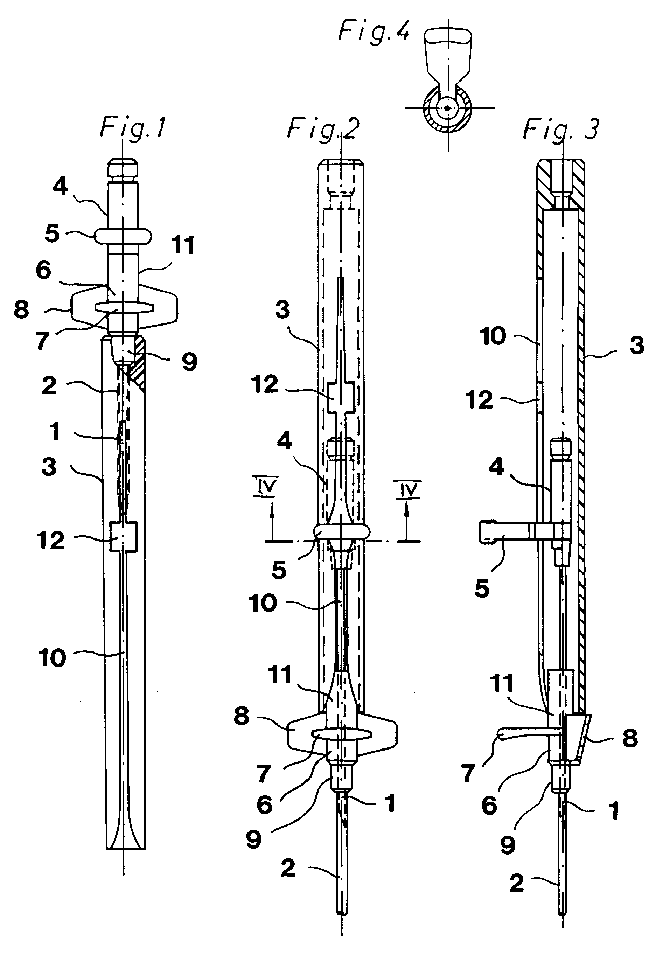

Gemäß der Darstellung in Fig. 1 sind eine Stahlkanüle 1 und eine darauf aufgeschobene Kunststoffkanüle 2 in einer zylindrischen Schutzhülse 3 untergebracht. Dabei verbleibt ein im wesentlichen zylindrischer proximaler Ansatz 4 der Stahlkanüle 1, der mit einer radial abstehenden Griffplatte 5 versehen ist, ebenso außerhalb der Schutzhülse 3 wie der wesentlichste Teil eines Ansatzes 6 der Kunststoffkanüle 2, der gleichfalls mit einer radial abstehenden Griffplatte 7 und zusätzlich mit einem tangential angeordneten Befestigungsflügel 8 versehen ist. Lediglich mit einem Teilstück 9 ragt der Ansatz 6 in die Schutzhülse 3 hinein. Der Außendurchmesser des Teilstücks 9 ist dem Innendurchmesser der aus elastisch verformbarem Kunststoff gefertigten Schutzhülse 3 so angepaßt, daß ein Klemmsitz erzielt wird. In diesem Zustand bilden die Teile eine kompakte Verkaufseinheit, die sich in konventioneller Weise steril verpacken läßt.

Gemäß den Darstellungen in den Fig. 2 und 3 dient die Schutzhülse 3 nach der Benutzung der Stahlkanüle zu deren Aufnahme, um sie schließlich ohne die Gefahr, sich durch die Stahlkanüle zu verletzen und zu infizieren, entsorgen zu können.

Die Schutzhülse 3 ist dazu mit einem Längsschlitz 10 versehen, der bis zu dem Ende der Schutzhülse 3 reicht, das in der Darstellung gemäß Fig. 1 von den Kanülen 1 und 2 weg weist und in der Darstellung gemäß den Fig. 2 und 3 auf ein zylindrisches Ansatzstück 11 der Kunststoffkanüle 2 aufgeschoben ist. An diesem Ende der Schutzhülse 3 ist der Längsschlitz 10 aufgeweitet, so daß sich ein Verbindungsbereich zwischen der Griffplatte 5 und dem Ansatz 4 der Stahlkanüle 1 mühelos in den Längsschlitz 10 einführen läßt, während die Schutzhülse 3 bis auf das zylindrische Ansatzstück 11 der Kunststoffkanüle 2 aufgeschoben ist. Unter vorübergehender Aufweitung des ansonsten nahezu geschlossenen Längsschlitzes 10 läßt sich sodann mit Hilfe der Griffplatte 5 die Stahlkanüle 1 in die Schutzhülse 3 hineinziehen, bis der Verbindungsbereich zwischen der Griffplatte 5 und dem Ansatz 4 der Stahlkanüle 1 in eine stufenförmige Verbreiterung 12 des Längsschlitzes 10 einrastet. Der Längsschlitz 10 nimmt dann infolge der Elastizität des Kunststoffmaterials, aus dem die Schutzhülse 3 gefertigt ist, seine Ursprungsbreite ein und verhindert eine Rückverschiebung der Stahlkanüle 1.

Es versteht sich, daß die Verbreiterung 12 so plaziert ist, daß die Stahlkanüle 1 spätestens dann vollständig von der Schutzhülse 3 umschlossen ist, wenn die Griffplatte 5 die Verbreiterung 12 erreicht hat. Sodann kann die Schutzhülse 3 vom Ansatzstück 11 der Kunststoffkanüle 2 abgezogen und mit der Stahlkanüle 1 gefahrlos entsorgt werden.

## Patentansprüche

1. Aufnahmevorrichtung aus einer mit einem Längsschlitz versehenen Schutzhülse (3) zur Unterbringung einer Stahlkanüle (1), die an ihrem proximalen Ende mit einem Ansatz versehen ist, an den ein radial vorstehendes Betätigungselement (5) angeformt ist, dadurch gekennzeichnet, daß der Innendurchmesser der Schutzhülse (3) dem Außendurchmesser eines zylindrischen Ansatzstücks (11) einer auf die Stahlkanüle (1) aufgeschobenen Kunststoffkanüle (2) derart angepaßt ist, daß sich unter geringfügiger Aufweitung der Längsschlitze (10) der Schutzhülse (3) diese auf das Ansatzstück (11) aufklemmen läßt, und der elastisch aufweitbare Längsschlitz (10) der Schutzhülse (3) bis zu ihrem auf das Ansatzstück (11) aufgesetzten Ende reicht, so daß beim Aufsetzen der Schutzhülse (3) auf das Ansatzstück (11) das Betätigungselement (5) in den Längsschlitz (10) eindringt und mit der Stahlkanüle (1) so weit verlagerbar ist, bis diese vollständig innerhalb der Schutzhülse (3) untergebracht ist.

2. Aufnahmevorrichtung nach Anspruch 1, dadurch gekennzeichnet, daß der Längsschlitz (10) am auf das Ansatzstück (11) aufsetzbaren Ende der Schutzhülse (3) durch dreiecksförmige Aussparungen im Hülsenmantel verbreitert ist.

3. Aufnahmevorrichtung nach Anspruch 1 oder 2, dadurch gekennzeichnet, daß der Längsschlitz (10) in einem Abstand vom auf das Ansatzstück (11) aufgesetzten Ende der Schutzhülse (3), der mindestens dem Abstand zwischen der Spitze der Stahlkanüle (1) und dem Betätigungselement (5) entspricht, eine radiale Verbreiterung (12) aufweist, in welche das Betätigungselement (5) einrastbar ist.

4. Aufnahmevorrichtung nach mindestens einem der Ansprüche 1 bis 3, dadurch gekennzeichnet, daß der Innendurchmesser der Schutzhülse (3) an seinem dem auf das Ansatzstück (11) aufgesetzten Ende gegenüberliegenden, gleichfalls offenen Ende dem Außendurchmesser eines zylindrischen Ansatzteils (9) der auf die Stahlkanüle (1) aufgeschobenen Kunststoffkanüle (2) entspricht, welches dem distalen Ende der Kunststoffkanüle (2) zugewandt ist.

## Claims

1. Retaining device consisting of a protective sleeve (3) fitted with a longitudinal slot to hold a steel cannula (1) which at its proximal end is fitted with an attachment onto which is moulded a radially projecting operating element (5), characterised in that the internal diameter of the protective sleeve (3) is sized to fit the external diameter of a cylindrical attachment piece (11) of a plastic cannula (2) pushed onto the steel cannula (1) such that under a slight expansion of the longitudinal slot (10) of the protective sleeve (3) this can be clamped onto the attachment piece (11), and the elastically expandable longitudinal slot (10) of protective sleeve (3) extends to its end which mounts on attachment piece (11) such that when the protective sleeve (3) is placed onto the attachment piece (11), the operating element (5) penetrates the longitudinal slot (10) and can be moved with steel cannula (1) until this is completely inserted in the protective sleeve (3).

2. Retaining device according to Claim 1, characterised in that the longitudinal slot (10) is enlarged at the end of the protective sleeve (3) which mounts on the attachment piece (11) by triangular cut-outs in the sleeve shell.

3. Retaining device according to Claim 1 or Claim 2, characterised in that at a distance from the end of the protective sleeve (3) which mounts on attachment piece (11), the longitudinal slot (10) has a radial expansion (12) in which the operating element (5) can be engaged, the said distance corresponding at least to the distance between the tip of the steel cannula (1) and the operating element (5).

4. Retaining device according to at least one of Claims 1 to 3, characterised in that the internal diameter of the protective sleeve (3), at its open end opposite the end which mounts the attachment piece (11), corresponds to the external diameter of a cylindrical attachment part (9) of the plastic cannula (2) pushed onto the steel cannula (1), which attachment part faces the distal end of the plastic cannula (2).

## Revendications

1. Dispositif de réception, constitué d'un manchon de protection (3) munie d'une fente longitudinale, destinée à la réception d'une canule (1) en acier munie à son extrémité proximale d'une pièce de prolongement sur laquelle est formé un élément d'actionnement (5) faisant saillie radialement, caractérisé en ce que le diamètre intérieur du manchon de protection (3) est adapté au diamètre extérieur d'une pièce de prolongement (11) cylindrique d'une canule (2) en matière plastique enfilée sur la canule (1) en acier, de façon à pouvoir, en élargissant légèrement la fente longitudinale (10) du manchon de protection (3), le bloquer sur la pièce de prolongement (11), et la fente longitudinale (10) du manchon de protection (3) qui est susceptible de s'élargir élastiquement, s'étend jusqu'à l'extrémité de celui-ci enfilée sur la pièce de prolongement (11) de façon telle que, lors de la mise en place du manchon de protection (3) sur la pièce de prolongement (11), l'élément d'actionnement (5) pénètre dans la fente longitudinale (10) et peut être déplacé en même temps que la canule en acier jusqu'à ce que celle-ci soit logée complètement dans le manchon de protection (3).

2. Dispositif de réception selon la revendication 1, caractérisé en ce que la fente longitudinale (10) est élargie à l'extrémité susceptible d'être enfilée sur la pièce de prolongement (11) au moyen d'échancrures triangulaires pratiquées dans l'enveloppe du manchon.

3. Dispositif de réception selon la revendication 1 ou 2, caractérisé en ce que la fente longitudinale (10) présente à une distance par rapport à l'extrémité du manchon de protection (3), mise en place sur la pièce de prolongement (11), qui correspond au moins à l'écart entre la pointe de la canule (1) en acier et l'élément d'actionnement (5), un élargissement (12) dans lequel l'élément d'actionnement (5) peut être encliqueté.

4. Dispositif de réception selon au moins une des revendications 1 à 3, caractérisé en ce que le diamètre intérieur du manchon de protection (3) correspond, à son extrémité également ouverte située à l'opposé de l'extrémité mise en place sur la pièce de prolongement (11), au diamètre extérieur d'une pièce de prolongement (9) de la canule (2) en matière plastique enfilée sur la canule (1) en acier, laquelle pièce de prolongement (9) est tournée vers l'extrémité distale de la canule (2) en matière plastique.
